# EUROPÄISCHE PATENTSCHRIFT

(11) **EP 4 267 043 B1**
(45) Veröffentlichungstag und Bekanntmachung des Hinweises auf die Patenterteilung: **12.11.2025**
(21) Anmeldenummer: 21835222.7
(22) Anmeldetag: 08.12.2021
(51) Int. Cl.: A61F 2/44, A61F 2/30, A61F 2/46

(54) **HÖHENVERSTELLBARES IMPLANTAT**
HEIGHT-ADJUSTABLE IMPLANT
IMPLANT À HAUTEUR RÉGLABLE

(30) Priorität: 22.12.2020 DE 102020007873
(43) Veröffentlichungstag der Anmeldung: 01.11.2023
(73) Patentinhaber: Taurus GmbH & Co. KG, 63755 Alzenau (DE)
(72) Erfinder: BLEISTEIN, Frank, 63755 Alzenau (DE); JAQUES, Annette, 63755 Alzenau (DE)
(74) Vertreter: Leinweber & Zimmermann
(86) Internationale Anmeldenummer: PCT/EP2021/084844
(87) Internationale Veröffentlichungsnummer: WO 2022/135940

(56) Entgegenhaltungen:
- EP-B1- 1 694 257
- US-A1- 2017 079 807
- US-A1- 2019 125 546

## Beschreibung

Die Erfindung betrifft Implantate, insbesondere Wirbelsäulenimplantate, die höhenverstellbar sind, also deren mit einem unteren Wirbelbereich in Eingriff stehendes Teil gegenüber seinem mit einem oberen Wirbelbereich in Eingriff stehenden Teil auf unterschiedliche axiale Relativlagen einstellbar ist. Insbesondere betrifft die Erfindung Implantate gemäß dem Oberbegriff von Anspruch 1.

Bei diesen ist die Höhenverstellbarkeit durch um eine Axialachse ausgeführte Drehung eines Antriebsteils bewirkbar. Das Antriebsteil ist axial gegenüber einem ersten Stützteil abgestützt und mit einem Außengewinde sowie mit einer Verzahnung versehen. Das Außengewinde wirkt mit einem Innengewinde eines gegenüber dem ersten Stützteil axial beweglichen aber rotatorisch nicht beweglichen zweiten Stützteil zusammen. Die Verzahnung ist an einer zum ersten Stützteil weisenden Axialseite des Antriebsteils angeordnet und durch einen durch das erste Stützteil radial hindurchführenden Zugang zugänglich.

Derartige Implantate, deren Höhenverstellbarkeit praktisch stufenlos ist, sind aus der Technik bereits bekannt, beispielsweise aus EP 1 694 257 B1.

Es hat sich jedoch herausgestellt, dass diese eingangs genannte Art der Höhenverstellbarkeit keine zuverlässig reproduzierbare Belastungswiderstandsfähigkeit aufweist. Selbst eine Weiterbildung der EP 1 694 257 B1 durch die Lehre der EP 2 055 269 B1 schafft diesbezüglich noch keine zufriedenstellende Verbesserung. Nicht zuletzt deshalb sind am Markt andere höhenverstellbare Implantate vertreten, bei denen das Antriebsteil mit einem Innengewinde ausgestattet ist und in einem u.a. einen einfacheren Zugang erlaubenden Weise in Projektion auf eine Ebene orthogonal zur Axialachse gesehen sowohl das erste Stützteil als auch das mit einer passenden Außenverzahnung versehene zweite Stützteil umschließt, ebenfalls im Bereich deren in Lücken zwischen jeweiligen Flanschen eingreifenden Bereiche. Derartige Implantate sind beispielsweise in EP 1 501 453 B1 beschrieben.

Der Erfindung liegt die Aufgabe zugrunde, ein gattungsgemäßes höhenverstellbares Implantat, bei dem die Höhenverstellbarkeit über ein gewindebehaftetes, in ein Gegengewinde eingreifendes Antriebsteils vermittelt wird, mit Blick auf eine gute Kombination aus zufriedenstellender Kompaktheit und Belastungsfähigkeit weiterzubilden.

US 2019/0125546 offenbart ein höhenverstellbares Implantat mit einem abgestützten Antriebsteil. US 2017/0079807 A1 offenbart ein höhenverstellbares Implantat, bei dem das Antriebsteil mit einer Innenverzahnung versehen und an einer Gehäuseschulter abgestützt ist.

Die Aufgabe wird in vorrichtungstechnischer Hinsicht durch ein Implantat mit den Merkmalen von Anspruch 1 gelöst. Die Abstützung des Antriebsteils erfolgt über eine einen bezüglich der Axialachse azimutal zwischen beiden Seiten des Zugangs gelegenen Raumbereich überspannende Brücke, und ein azimutal im Bereich des Zugangs und bezüglich der Axialachse radial weiter innen als die Verzahnung gelegener Abstützbereich weist die Brücke auf.

So wurde im Rahmen der Erfindung herausgefunden, dass herkömmliche Wirbelsäulenimplantate der eingangs genannten Art statischen Belastungstests zwar ausreichend zuverlässig standhalten, bei dynamischen rotatorischen Belastungstests jedoch periodisch Kippmomente erzeugt werden, die zu einer Beschädigung bis hin zu einem Materialbruch am Implantat führen.

Bei der erfindungsgemäßen Lösung wird dagegen derartigen Kippmomenten durch den (in Projektion auf die Ebene orthogonal zur Axialachse gesehen) azimutal im Bereich des Zugangs und radial weiter innen als die Verzahnung gelegenen Abstützbereich der Abstützung des Antriebsteils entgegengewirkt und dadurch ein nicht nur gegenüber statischen, sondern auch dynamischen Belastungen widerstandsfähiges Implantat geschaffen.

Erfindungsgemäß erfolgt die Abstützung somit über eine einen zwischen beiden Seiten einer azimutalen Begrenzung des Zugangs gelegenen Raumbereich überspannende Brücke. Es versteht sich, dass diese Brücke radial nicht in den Bereich hineinragt, in dem die Verzahnung für einen Verzahnungseingriff mit einer radialen Antriebswelle zugänglich ist. Auflagelasten können jedenfalls flächig aufgenommen und dann wenigstens teilweise über die beiden azimutalen Begrenzungen abgeleitet werden. Wie weiter unten mit Bezug auf die Figuren erläutert ist, weisen die Zähne der Verzahnung des Antriebsteils von dem Außengewinde des zweiten Stützteils weg. Entsprechend ist der Verzahnungseingriff zwischen der Verzahnung und einer rotierenden Gegenverzahnung beim in Drehung Versetzen der Verzahnung bezüglich der Drehachse der Gegenverzahnung zur Seite der Außenverzahnung hin gelegen, also an der zum zweiten Stützteil weisenden Axialseite des Zugangs.

In einer bevorzugten Ausgestaltung erstreckt sich ein Öffnungsbereich des Zugangs bis zu einem Axialdurchgang durch einen Axialbereich des ersten Stützteils, das von einer drehfest mit dem Antriebsteil gekoppelten Axialwelle durchdrungen ist. Dies erlaubt eine synergetische Nutzung des Zugangs zum einen um durch Betätigung des Antriebs die Höhenverstellbarkeit zu bewirken, zum anderen, um einen Zugang für eine Verstellsicherung zu erhalten. So ist in diesem Zusammenhang eine das Implantat im Zustand eingestellter Höhe in einer Sicherungsstellung gegen eine Verdrehung des Antriebsteils gegenüber dem ersten Stützteil sichernde, durch den Öffnungsbereich tretende Sicherung vorgesehen.

In einer bevorzugten Ausgestaltung ist die Brücke durch ein von dem ersten Implantatteil separates Abstützteil gebildet. Dies erleichtert die Herstellung trotz eines zusätzlichen Bauteils. Das Antriebsteil stützt sich zur axialen Seite, zu der die Zahnköpfe seiner Verzahnungen weisen, an der Brückeldem separaten Bauteil ab. Wie weiter unten mit Bezug auf die Figuren erläutert ist, können Zähne der Verzahnung radial weiter innen als am Verzahnungseingriff mit ihren Zahnköpfen auf dem separaten Abstützteil aufliegen.

In einer bevorzugten Ausgestaltung erstreckt sich das Abstützteil azimutal über mehr als 60°, bevorzugt mehr als 90°, weiter bevorzugt über mehr als 120°, insbesondere mehr als 180°, und ist insbesondere ringförmig gebildet. Dadurch wird eine nochmals gleichmäßigere Lastverteilung erreicht.

In einer weiteren bevorzugten Ausführungsform ist vorgesehen, dass das Abstützteil auf einer radial zwischen der Verzahnung und dem Axialdurchgang gelegenen, in der Ebene orthogonal zur Axialrichtung insbesondere kontinuierlich und insbesondere planar gebildeten Auflagefläche des ersten Stützteils aufliegt. Diese Auflagefläche kann die dem Antriebsteil zugewandte Axialfläche eines Versteifungskörpers sein, der Axialvorsprünge bzw. Axialwände des ersten Stützteils, die mit Axialvorsprüngen/Axialwänden des zweiten Stützteils kämmen, untereinander verbindend stützt. Dieser Verstärkungskörper kann insbesondere einstückig mit dem ersten Stützteil gebildet sein, in einer bevorzugten Variante den Zugang an einer dem Antriebsteil abgewandten Axialseite begrenzen.

In einer weiteren bevorzugten Ausführungsform ist ein Ringspalt zwischen der Verzahnung und der Auflagefläche vorgesehen. Das heißt, die mit einer Verzahnung einer radialen Antriebswelle kämmende Verzahnung des Antriebsteils, d.h. die im Eingriffsbereich liegenden Zahnköpfe der Verzahnung sind an der Abstützung nicht beteiligt. Dies entlastet die erforderliche Axialpräzision bei Herstellung des Implantats.

Zudem ist in einer bevorzugten Ausgestaltung eine in einem dem ersten Stützteil zugewandten axialen Endbereich des Innengewindes des zweiten Stützteils dieses Innengewinde sperrende Axialsicherung vorgesehen. Diese kann beispielsweise in Form eines einen der kämmenden Vorsprünge/Axialwände des zweiten Stützteils radial durchdringenden Stift verkörpert sein, der bis in den radialen Erstreckungsbereich des Innengewindes hineinragt. Der Stift kann nach dem Einsetzen fest mit dem zweiten Stützteil verbunden werden, beispielsweise durch Punktschweißen.

In einer weiteren bevorzugten Gestaltung können das erste Stützteil und/oder das zweite Stützteil insbesondere plattenförmige freie axiale Endbereiche haben, deren Flächenausdehnung im Querschnitt gesehen jedes Innengewinde um mehr als 10%, bevorzugt mehr als 20%, weiter bevorzugt mehr als 40%, insbesondere mehr als 60% übersteigt. Bei diesen axialen Endbereichen handelt es sich bevorzugt um z.B. mittels einer Schraube endseitig angeschraubten separaten Teilen. Dies erlaubt das Heranziehen der gleichen Grundstruktur mit dem Mechanismus zur Höhenverstellbarkeit für unterschiedliche Applikationen, indem diese Endbereiche in mehrfacher Ausgestaltung vorbereitet und wahlweise angekoppelt werden können.

In einer weiteren bevorzugten Ausgestaltung weist das erste Stützteil im Bereich des radialen Zugangs eine einen vorragenden, insbesondere diesen umschließenden und dabei durch seine Außenkontur einen insbesondere als Drehsperre gebildeten Formsitz für eine Komplementärform eines Bedieninstruments bildenden Vorsprung auf. Dies erleichtert die Bedienung, indem die Erstkopplung zwischen Bedieninstrument und erstem Stützteil nicht über beispielsweise ein Einschrauben erfolgen muss.

In diesem Zusammenhang ist auch ein Instrument zur Betätigung des Implantats vorgesehen, mit einer von Hand betätigbaren ersten Welle mit einer Gegenverzahnung zu der Verzahnung des Antriebsteils an ihrem proximalen Ende.

Das Instrument ist bevorzugt mit einer Hülsenanordnung ausgestattet, durch die die erste Radialwelle führbar ist, und einer zweiten Radialwelle mit einer axial lösbaren drehfesten Kopplung zur Axialsicherung, wobei insbesondere auch die Axialsicherung durch das Innere der Hülsenanordnung passt. Die Axialsicherung kann beispielsweise in Form einer Schraube gebildet sein und wird mit der zweiten Radialwelle durch die Hülsenanordnung eingeführt und in ein Innengewinde im Zugang eingeschraubt, so dass deren proximales Ende bis zum Axialdurchgang eingeschraubt die Axialwelle blockieren kann.

In einer weiteren bevorzugten Ausgestaltung weist die Hülsenanordnung eine Außenhülse mit, an ihrem proximalen Ende, Komplementärform zum Formsitz des Vorsprungs und eine Innenhülse mit einem Gewinde zum Verschrauben mit einem Innengewinde der Zugangsöffnung auf.

Des Weiteren stellt die Erfindung bereit ein Set, aufweisend ein oder mehrere Implantate nach einem der obigen Gesichtspunkte und einem Instrument nach einem der obigen Gesichtspunkte.

Bevorzugt ist vorgesehen, dass das Set mehrere Implantate mit wenigstens zwei oder mehr unterschiedlichen Axialabmessungen der Implantate bei Einstellung auf deren geringste einstellbare Höhe aufweist. Weiter ist vorgesehen, dass das Set wenigstens zwei Paare der axialen Endbereichteile unterschiedlicher Flächenausdehnung beinhaltet.

In einer bevorzugten Ausgestaltung weisen eine oder mehrere der axialen Endbereiche, Schrauben zu deren Anbringung, Antriebsteil, Axialverlängerung des Antriebsteils, einen bezüglich der Radialrichtung zentralen axialen Durchgang auf. Dies dient der Gewichtsverlagerung des Implantats.

Das Material ist aus einem für die Anwendung als Implantat grundsätzlich geeigneten und zugelassenen Material gebildet, bevorzugt aus einem metallischen Material, insbesondere aus Titan oder einer Titan-Legierung.

Weitere Merkmale, Einzelheiten und Vorteile der Erfindung ergeben sich aus der nachfolgenden Beschreibung mit Bezug auf die beigefügten Figuren, von denen
Fig. 1 ein Wirbelsäulenimplantat in einer perspektivischen Ansicht zeigt,
Fig. 2 eine teilweise im Axialschnitt gehaltene Darstellung des Wirbelsäulenimplantats ist,
Fig. 3 eine vereinfachte, teilweise im Axialschnitt dargestellte Ansicht einer Grundeinheit des Wirbelsäulenimplantats zeigt,
Fig. 4 schematische Perspektivdarstellungen eines Bereichs einer Axialseite eines unteren Teils der Grundeinheit des Wirbelsäulenimplantats aus zwei unterschiedlichen Blickwinkeln ist,
Fig. 5 einen Teil des Antriebs mit Außengewinde und Verzahnung für einen eine Höhenverstellung auslösenden Verzahnungseingriff zeigt, a) von schräg oben, b) schräg von unten ohne und c) mit einer Abstützkraftaufnahme,
Fig. 6 eine Verriegelungsschraube zur Sicherung einer eingestellten Implantathöhe zeigt,
Fig. 7 Bestandteile eines Setzinstrumentes für das Implantat zeigt,
Fig. 8 deren Ankopplungsende in zwei unterschiedlichen Betriebszuständen zeigt,
Fig. 9 die Kopplung von Implantat und Setzinstrument in zwei verschiedenen Betriebszuständen zeigt,
Fig. 10 einen Satz von Wirbelsäulenimplantaten unterschiedlicher axialer Mindesthöhe zeigt.

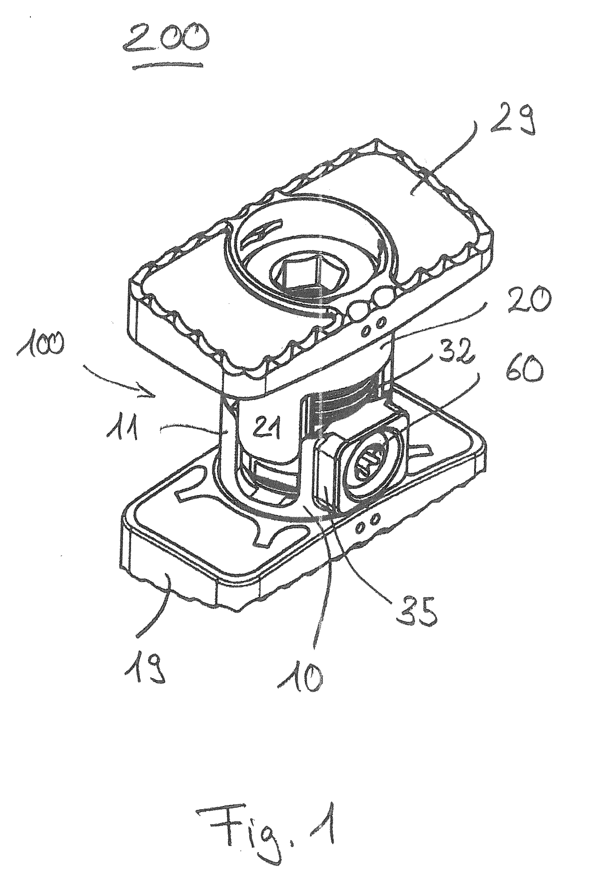

In Fig. 1 ist in schematischer Darstellung ein Wirbelsäulenimplantat 200 dargestellt, welches in einem für den Fachmann geläufigen Weise zwischen zwei Wirbeln einsetzbar ist und der Stützung der Wirbelsäule dient.

In diesem Ausführungsbeispiel stützt sich das Implantat 200 mit plattenförmigen Endplatten (untere Endplatte 19 und obere Endplatte 29) an den Wirbeln ab, welche mit einer Grundeinheit 100 verschraubt sind. Die genaue Form und Gestalt wie auch Struktur der Endplatten ist jedoch nicht auf die figürlich dargestellte Rechteckform eingeschränkt, vielmehr könnten die Platten auch eine andere Form und/oder Struktur aufweisen, etwa oval oder kreisförmig. Die Endplatten können beispielsweise mit einem strukturierten Gitter, aus einer Titanlegierung gesintert, versehen sein.

Das Implantat 200 ist höhenverstellbar dahingehend, dass ein unteres Teil 10 der Grundeinheit 100 gegenüber einem oberen Teil 20 der Grundeinheit 100 axial verschieblich ist. Die Verschieblichkeit weist eine Axialführung auf, die im vorliegenden Ausführungsbeispiel dadurch gestaltet ist, dass formschlüssig ineinandergreifende obere Flanschbereiche 21 in komplementäre Lücken zwischen unteren Flanschbereichen 11 des unteren Teils 10 eingreifen und vice versa, wie in Fig. 1 gut erkennbar.

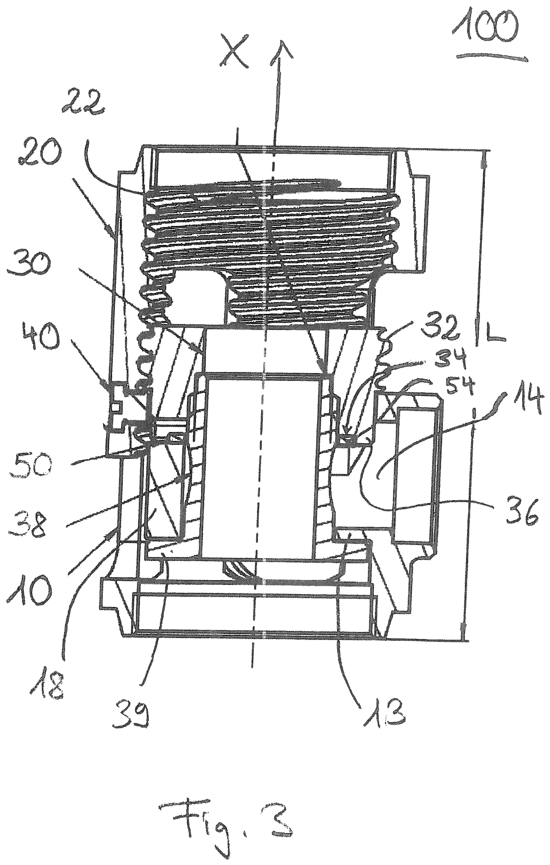

In einer bevorzugten Gestaltung sind wie im dargestellten Ausführungsbeispiel realisiert drei Flanschbereiche 11 und drei Flanschbereiche 21 vorgesehen, die den azimutalen Gesamtteil von 2π bzw. 360° untereinander aufteilen. Entsprechend einer bevorzugten, jedoch nicht zwingenden Gestaltung der Erfindung ist wie im Ausführungsbeispiel von Fig. 1 gezeigt ein mit einer radialen Zugangsöffnung 14 (Fig. 3, in Fig. 1 sperrt Schraube 60 die Öffnung 14) versehener Flansch mit einer Azimutalerstreckung von mehr als 60° gebildet, und die übrigen zwei unteren Flansche 11 und drei oberen Flansche 21 belegen den verbleibenden azimutalen Teil des azimutalen Gesamtumfangs.

Der Mechanismus der Höhenverstellbarkeit des Implantats 200 bzw. der Grundeinheit 100 mit dem unteren Teil 10 und dem oberen Teil erfolgt gemäß einem dem Fachmann aus der Technik grundsätzlich bereits bekannten Verstellmechanismus, bei dem nämlich das obere Teil 20 mit einem Innengewinde 22 versehen ist, in welches das Außengewinde 32 eines Antriebsrads 30 eingreift. Dreht sich das Antriebsrad 30 bei nicht drehendem unteren Teil 10, schiebt sich das gegenüber dem unteren Teil 10 zwar axial verlagerbare, aber nicht drehbare obere Teil 21 je nach Drehrichtung nach oben (Expansion) bzw. nach unten (Kontraktion).

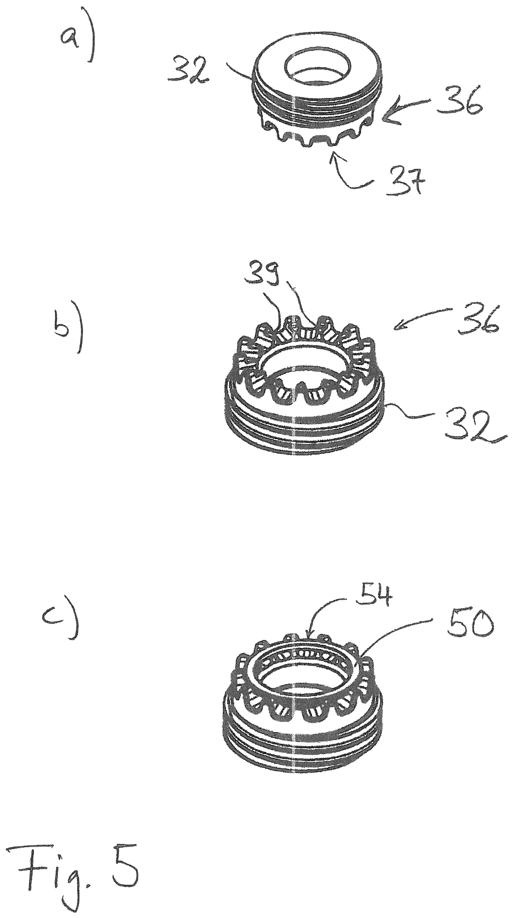

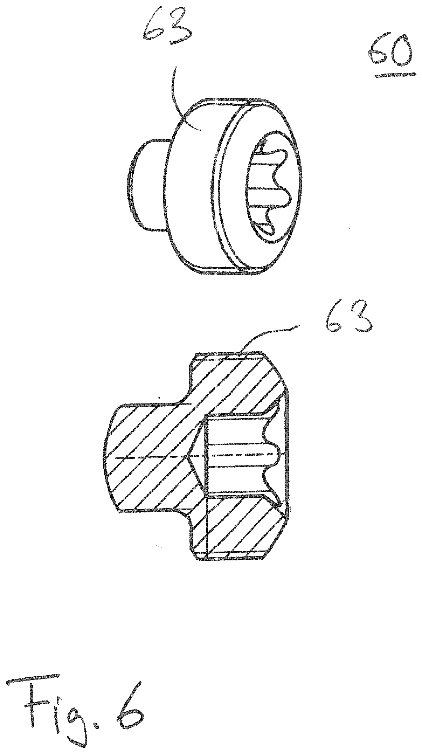

Um das Antriebsrad 30 zu verdrehen, weist dieses einen Zahnkranz 36 (Fig. 3 und Fig. 5a, c) auf, und zwar an seiner nach unten weisenden Axialseite am radial äußeren Rand. Auf diese Weise kann das Antriebsrad 30 durch eine um eine Achse einer Welle eines Betätigungsinstruments rotierbare Gegenverzahnung zur Verzahnung 36 in Drehung versetzt werden. Durch die Öffnung 14 wird der hierzu erforderliche Zugang geschaffen (Fig. 3), in der Darstellung von Fig. 1 ist der Zugang durch eine Verriegelungsschraube 60 blockiert. Die Verriegelungsschraube 60 (Fig. 6) wird eingesetzt, nachdem die gewünschte Höhe des Implantats 200 bzw. der Grundeinheit 100 eingestellt wurde und hält eine drehfest mit dem Antriebsrad 30 angekoppelte Axialverlängerung 38 des Antriebsrads 30 durch Schraubklemmung gegenüber dem unteren Teil 10 drehfest.

Das untere Teil 10 weist einen, wie in diesem Ausführungsbeispiel bevorzugt mit den Flanschbereichen 11 hier einstückig gebildeten Verstärkungsbereich 18 auf, der radial gesehen an die Flanschbereiche 11 nach innen anschließt und somit erlaubt, dass die Flansche 21 des zweiten Teils ungehindert axial an ihm vorbei verschoben werden können. Zudem erstreckt sich der Verstärkungsbereich 18 radial gesehen in den Bereich, in dem auch die Verzahnung 36 des Antriebsrads 30 liegt.

Um neben der radialen Zugänglichkeit zur Zuführung der Gegenverzahnung auch eine axiale Zugangsmöglichkeit zur Herstellung des Verzahnungseingriffs zum drehenden Antreiben des Antriebsrads 30 zu schaffen, weist der Verstärkungsbereich 18 eine in diesem Ausführungsbeispiel im Wesentlichen U-förmige Ausnehmung auf, und zwar azimutal gesehen im Bereich der Zugangsöffnung 14. Im Radialbereich der Verzahnung 36 weist der hier einstückig mit dem ersten Teil gebildete Verstärkungsbereich 18 somit eine Begrenzung des im Wesentlichen U-förmigen Ausschnitts in Form von axialen Seiten 15l und 15r und einem Boden 13 auf.

Radial nahezu jedoch innerhalb dieses Radialbereichs der Verzahnung 36 wird der Zwischenraum zwischen den Seiten 15l und 15r durch ein Kreisringsegment 54 eines Ringkörpers 50 überbrückt. Dabei liegt der Ringkörper 50 auf einem kreisringsegmentförmigen Auflagebereich einer axialen Seite 16 des Verstärkungsbereichs 18 auf, der sich azimutal an der der Zugangsöffnung 14 abgewandten Seite zwischen den Seiten 15l und 15r erstreckt.

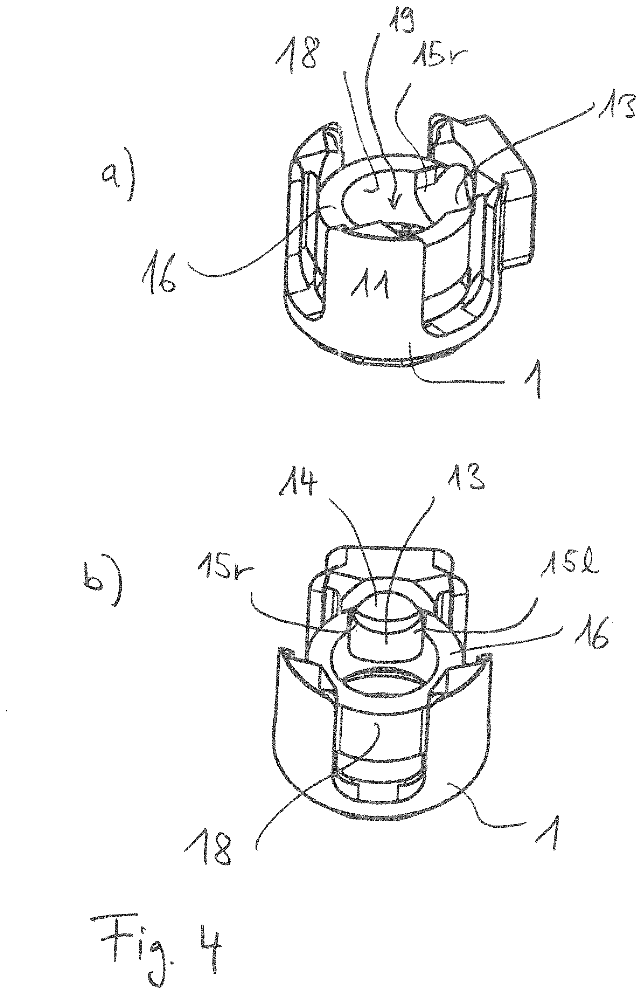

In Fig. 4a und 4b ist nochmals besser zu erkennen, dass bei aufgelegtem Ring 50 der kreissegmentförmige Bereich 54 des Rings 50 (Fig. 5b um 180° (oben-unten) verschwenkt auf Auflage 16 in Lage aus Fig. 4b) "aufgesetzt") die Öffnung unter Verbindung der azimutalen Begrenzungen 15l und 15r der Öffnung 14 brückenartig überspannen wird. In Fig. 5 ist die Verzahnung 36 nochmal in mehreren Ansichten auch von unten dargestellt. In der Darstellung von Fig. 5c ist gegenüber der Darstellung von Fig. 5b noch der Ring 50 mit dargestellt. Man erkennt, dass die radial außen gelegene Verzahnung für den Verzahnungseingriff bei der Herstellung auch mit größerer radialer Erstreckung erzeugt und dann radial innen die Zahnhöhe unter Bildung einer diskontinuierlichen Anlagefläche 39 für den Ring 50 reduziert wird.

Wie aus Fig. 3 erkennbar ist, stützt sich das Antriebsrad 30 axial über den die Brücke 54 aufweisenden Ring 50 an dem Verstärkungsbereich 18 des unteren Teils 10 ab.

Zudem ist die axiale Höhe des Rings 50 so bemessen, dass die Zahnköpfe 37 (Fig. 5a) der Verzahnung 36 selbst, jedenfalls in der bevorzugten Gestaltung wie auch im Ausführungsbeispiel dargestellt, nicht mehr auf der Fläche 16 aufliegen. Das heißt, es ist ein Spalt zwischen den Zahnköpfen 37 der Verzahnung 36 und der Auflage 16 vorgesehen. Demnach erfolgt die Ableitung der Abstützkraft der Abstützung des Antriebsrads 30 in das untere Teil 10 mittelbar über den Ring 50 und azimutal auch im Bereich der Öffnung 14.

In einer bevorzugten Gestaltung ist wenigstens der Brückenbereich 54 in Form eines gegenüber dem Verstärkungsbereichs 18 separaten Bauteils vorgesehen, weiter bevorzugt wie in z.B. Fig. 4 am besten dargestellt in Form eines geschlossenen Ringkörpers, der insgesamt als eigenständiges Bauteil vorgesehen ist.

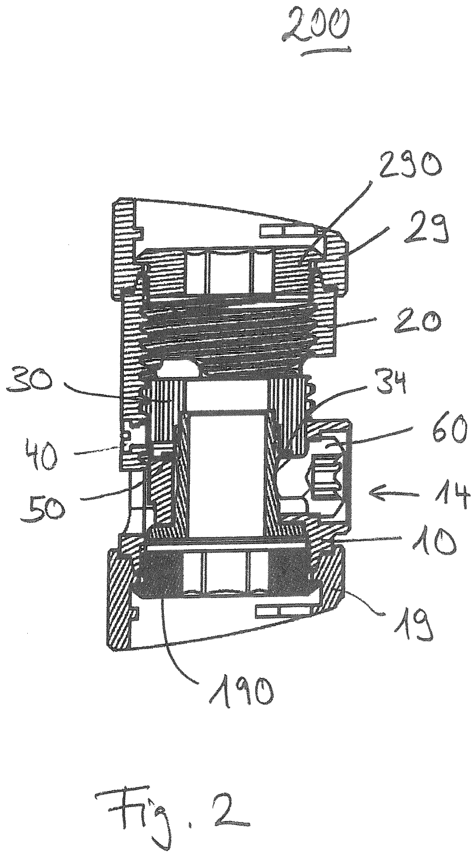

Wie weiter aus insbesondere Fig. 2 und Fig. 3 erkennbar ist, sorgt ein Stift 40, der in diesem Ausführungsbeispiel in der diametral entgegengesetzten Position wie die Zugangsöffnung 14 im unteren Endbereich der Innenverzahnung 22 vorgesehen ist und dieses Innengewinde 22 durchdringt, für einen axialen Stopper, bzw. Anschlag, der ein weiteres Verdrehen der Gewinde verhindert und somit die maximale Extraktion der Grundeinheit 100 bestimmt. Der Stift 40 stellt somit gleichzeitig eine Verliersicherung bereit.

Wie aus Fig. 1 erkennbar ist, verläuft die Grenzfläche zwischen den unteren Flanschen 11 und den oberen Flanschen 21 nicht eben, sondern weist im Querschnitt gesehen eine Stufe auf, so dass die oberen Flansche 21 in den Zwischenräumen zwischen den unteren Flanschen 11 schwalbenschwanzartig gehalten sind.

Der Verstärkungsbereich 18 ist wie bereits gesagt grundsätzlich ringförmig gestaltet und weist einen axialen Durchgang 19 auf, welcher von der als Hülsenschraube gebildeten axialen Fortsetzung 38 des das Außengewinde 32 tragenden Antriebsrads 30 durchdrungen ist. Dieser Fortsatz 38 weist an seinem von dem Antriebsrad 30 weg weisenden freien Ende einen Flanschbereich 39 auf, so dass die beim Höhenverstellen mit dem Antriebsrad 30 gedrehte Einheit den Verstärkungsbereich 38 axial gesehen umschließt und insgesamt über wenigstens einen überwiegenden Teil seiner Länge oder sogar, wie in dem bevorzugten Ausführungsbeispiel dargestellt, über seine gesamte axiale Länge einen radial zentralen Hohlraum aufweist. Auch die Befestigungsschrauben 190, 290 für die Endplatten 19 bzw. 29 sind bevorzugt mit einem radial gesehen zentralen Hohlraum gebildet. Zur Aufnahme der Befestigungsschrauben 190, 290 weist das erste Teil 10 und das zweite Teil 20 jeweils an seinem freien Ende ein mit dem Gewinde dieser Schrauben zusammenwirkendes Innengewinde auf.

Die Form des Innengewindes 22 im Axialschnitt gesehen hat eine im Wesentlichen in der Ebene orthogonal zur axialen Achse X liegende, zu dem freien Ende des oberen Teils 20 weisende, im Wesentlichen planare Flanke und eine schräg zu dieser Ebene verlaufende, von diesem freien Ende wegweisende Flanke. Der Steigungswinkel des Gewindes beträgt in diesem Ausführungsbeispiel etwa 1,75°.

Wie aus Fig. 1 zu erkennen ist, ist an dem unteren Flansch 11 mit der Zugangsöffnung 14 ein Vorsprung 35 in Form einer umlaufenden Wandstruktur gebildet, dessen Außenkontur einen Formsitz für das weiter unten beschriebene Setzinstrument bildet. Dieser Formsitz ist in diesem Ausführungsbeispiel von rechteckiger Grundform mit abgerundeten Ecken gebildet. Der Innenwandbereich des Vorsprungs 35 trägt ein Innengewinde, das mit einem Gewinde am freien Ende eines weiteren Teils des Setzinstruments zusammenwirkt (siehe unten).

Zudem hat die Zugangsöffnung 14 noch ein radial weiter innen liegendes Innengewinde, das mit einem Außengewinde der Verriegelungsschraube zusammenwirkt. Die Verriegelungsschraube 60 ist in Fig. 6 nochmals alleinstehend bildlich in einer Perspektivansicht dargestellt. Man erkennt einen Schraubkopfantrieb, in diesem Ausführungsbeispiel torähnlich mit π/3 Drehsymmetrie (es versteht sich, dass beliebige andere Schraubkopfantriebe denkbar sind), zur Ankopplung eines Teils eines Setzinstruments (siehe unten) am Kopf der Verriegelungsschraube, sowie eine ballige Gestaltung der distalen freien Stirnfläche der Schraube, für einen flächigen Kontakt mit der in Fig. 2 erkennbaren hohlballigen Gestaltung der axialen Fortsetzung 38 in einem Bereich auf Höhe der Zuführöffnung 14. Das Außengewinde der Verriegelungsschraube 60 ist mit dem Bezugszeichen 63 gekennzeichnet.

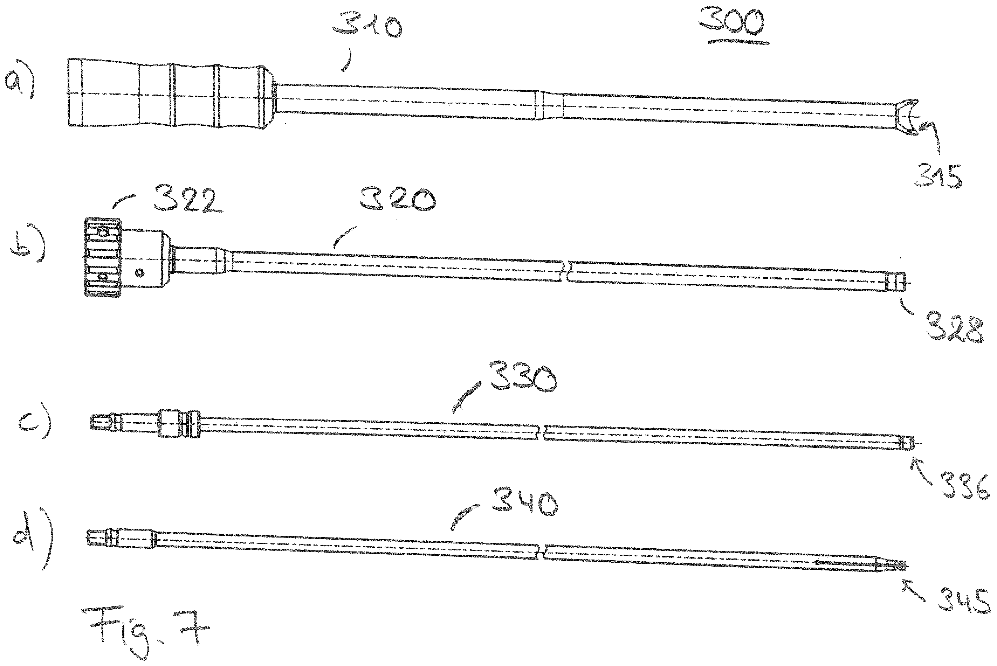

In den Figuren 7a bis 7f sind Teile eines mehrteiligen Setzinstrumentes 300 gezeigt, das mit dem erfindungsgemäßen Implantat zusammenwirkt.

Fig. 7a) zeigt in einer Ansicht eine Außenhülse 310, an deren proximalem operativen Ende ein Sitz 315 gebildet ist, der mit dem Sitz 35 des Implantats formschlüssig wirkt. Die Außenhülse 310 wird auf den Sitz 35 auf das Implantat aufgesteckt. Durch diese Außenhülse 310 kann die in Fig. 7b dargestellte Innenhülse 320 eingeführt werden. Diese hat an ihrem operativen proximalen Ende ein Außengewinde 328, welches mit dem Innengewinde in der Innenberandung des Vorsprungs 35 zusammenwirkt. Am distalen Ende ragt ein mit Griffrillen versehener Greifring radial über die Außenhülse hinaus und übergreift das distale Ende der Außenhülse.

Die distalen Enden der Wellen 330 und 340 dienen dem Aufsatz eines nicht figürlich gezeigten weiteren Teils des Setzinstruments, einem Palmgriff mit Drehmomentbegrenzer.

Innerhalb der Innenhülse können unterschiedlich gestaltete Wellen eingeführt werden, zum einen die in Fig. 3c gezeigte Distraktionswelle 330, an deren proximalem operativen Ende eine Verzahnung gebildet ist, nämlich eine Gegenverzahnung 336 zur Verzahnung 36, zum anderen in einem anderen Betriebszustand eine am operativen proximalen Ende mit passender Kopplung 345 zum Einschrauben der Verriegelungsschraube 60 ausgestatteten Verriegelungswelle 340, die in Fig. 7d gezeigt ist.

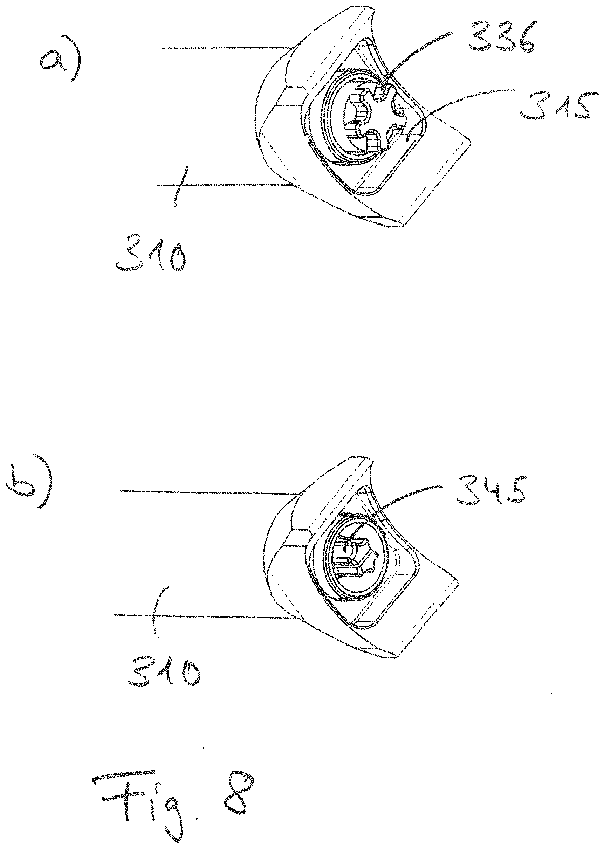

Die Gegenverzahnung 336 ist in Fig. 8a nochmals in vergrößerter Darstellung erkennbar, bei der das Setzinstrument in dem ersten Betriebszustand mit eingeführter Distraktionswelle mit ihrem proximalen Endbereich dargestellt ist. In Fig. 8b erkennt man dagegen die Ankopplung 345 passend zur Innengestaltung des Schraubkopfes der Verriegelungsschraube 60. In beiden Darstellungen erkennt man zudem das Außengewinde der Innenhülse 320, mit dem das Setzinstrument temporär mit dem Implantat verschraubt wird. Die Innenhülse 320 wirkt mit der Außenhülse 310 derart zusammen, dass bei Realisierung des Formschlusses zwischen dem Gegensitz 315 zu Sitz 35 eine automatische Zentrierung des Außengewindes 328 mit dem Innengewinde bewirkt.

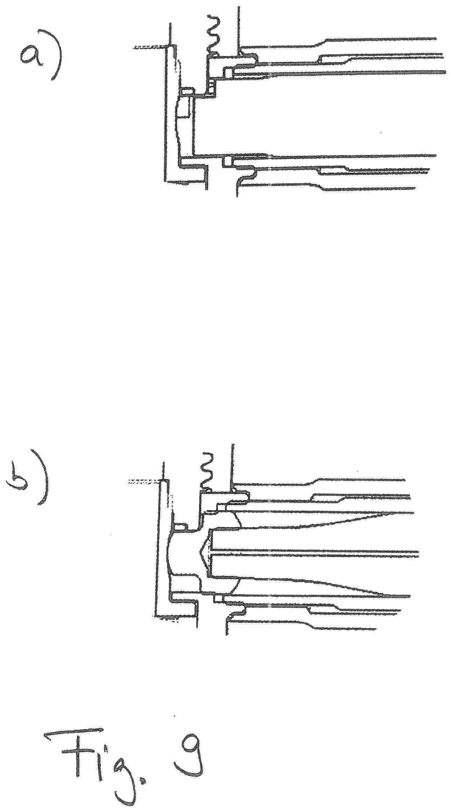

Ist im ersten Betriebszustand die Distraktionswelle 330 (Fig. 7c) in die Innenhülse 320 eingeführt, kommt diese durch eine Radialabstimmung radial auf Höhe der Verzahnung 36 zu liegen (Fig. 9a). Eine Drehung der Distraktionswelle 330 in Verzahnungseingriff des kämmenden Verzahnungspaars 36-336 um die radial zur axialen Achse X des Implantats verlaufenden Wellenachse dreht das Antriebsrad 30 und verschiebt über die Gewindekopplung 32-22 das obere Teil 20 axial gegenüber dem unteren Teil 10.

Ist die gewünschte axiale Höhe des Implantats auf diese Weise eingestellt, wird anstelle der Distraktionswelle 330 die Verriegelungswelle 340 in die Innenhülse 320 eingesetzt (zweiter Betriebszustand), die bereits die Verriegelungsschraube 60 aufgesteckt hat, und die durch die Innenhülse 320 eingeführte Verriegelungsschraube 60 wird eingeschraubt, und fixiert die Drehlage von Antriebsring 30 und unterem Teil 10 (Fig. 2, 9b).

Die gegenüber der Radialachse axiale Kopplungsfläche der Verriegelungsschraube 60 kann wie hier realisiert ballig ausgebildet sein, um mit einer ringförmig umlaufenden Hohlballigkeit der Axialfortsetzung 38 des Antriebsrings 30 mit einem größeren Flächenkontakt als einer kraftfrei gesehen nur Punktberührung anzuliegen.

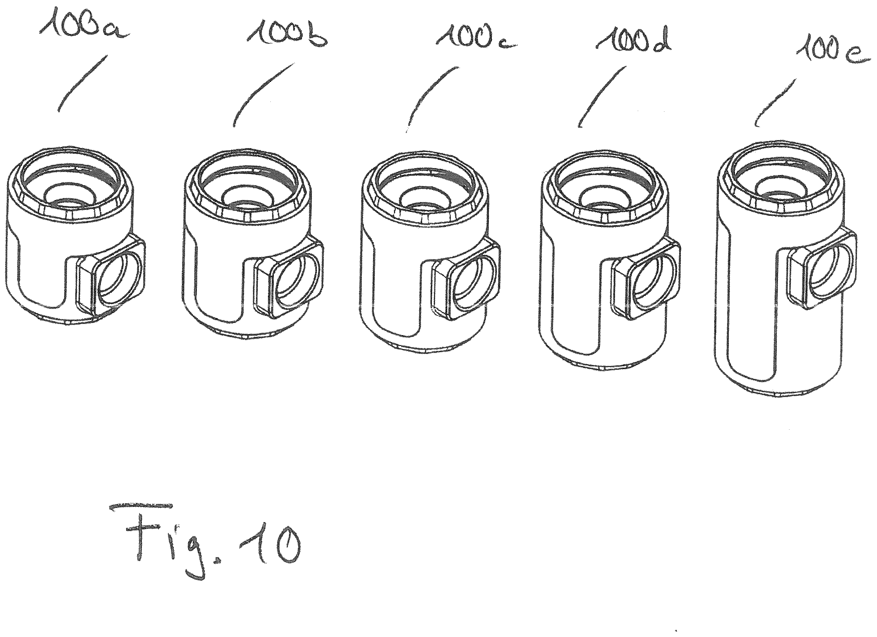

In Fig. 10 sind noch mehrere Grundkörper 100a, 100b, 100c, 100d und 100e dargestellt, die einem Wirbelsäulenimplantat-Set gemäß der Erfindung noch höhere Einsatzvariabilität verleihen. Man erkennt, dass die Implantate in ihrem (Null-)Zustand geringster axialer Ausdehnung eine unterschiedliche Axialausdehnung aufweisen. Die Flanschbereiche von erstem und zweitem Teil sind hierzu mit jeweils wachsender Länge ausgeführt, wobei die Position des Zugangs bezüglich des freien Endes des ihm zugeordneten Flansches gleich ist. Es versteht sich, dass das Set nicht auf eine Gestaltung mit fünf unterschiedlichen Implantathöhen eingeschränkt ist, es könnte auch eine andere Anzahl sein, auch ist die jeweils untereinander vorgesehene Höhenabstufung nicht auf die dargestellte eingeschränkt.

Des Gleichen ist auch sonst die Erfindung nicht auf die anhand der bevorzugten Ausführungsbeispiele dargestellten Einzelheiten eingeschränkt. Vielmehr können die einzelnen Merkmale der vorhergehenden Beschreibung sowie der nachstehenden Ansprüche einzeln und in Kombination für die Verwirklichung der Erfindung in ihren verschiedenen Ausführungsformen wesentlich sein.

## Patentansprüche

1. Implantat (200), insbesondere zum Stützen der Wirbelsäule, mit einer durch um eine Axialachse (X) ausgeführte Drehung eines axial gegenüber einem ersten Stützteil (10) abgestützten und mit einem mit einem Innengewinde (22) eines gegenüber dem ersten Stützteil axial beweglichen aber rotatorisch nicht beweglichen zweiten Stützteil (20) zusammenwirkenden Außengewinde (32) sowie an seiner zum ersten Stützteil weisenden Axialseite mit einer durch einen durch das erste Stützteil radial hindurchführenden Zugang (14) zugänglichen Verzahnung (36) versehenen Antriebsteils bewirkbaren Höhenverstellbarkeit,
**dadurch gekennzeichnet, dass** die Abstützung des Antriebsteils über eine einen bezüglich der Axialachse (X) azimutal zwischen beiden Seiten des Zugangs gelegenen Raumbereich überspannende Brücke (54) erfolgt und das Implantat (200) einen azimutal im Bereich des Zugangs und bezüglich der Axialachse (X) radial weiter innen als die Verzahnung gelegenen sowie die Brücke (54) aufweisenden Abstützbereich der Abstützung aufweist.

2. Implantat nach Anspruch 1, bei dem sich ein Öffnungsbereichs des Zugangs bis zu einem Axialdurchgang durch einen Axialbereich des ersten Stützteils erstreckt, das von einer drehfest mit dem Antriebsteil gekoppelten Axialstelle (38) durchdrungen ist.

3. Implantat nach Anspruch 2, mit einer das Implantat im Zustand eingestellter Höhe in einer Sicherungsstellung gegen eine Verdrehung des Antriebsteils gegenüber dem ersten Stützteil sichernden, durch den Öffnungsbereich tretenden Sicherung (60).

4. Implantat nach einem der Ansprüche 1 bis 3, bei dem die Brücke (54) durch ein von dem ersten Implantatteil separates Abstützteil (50) gebildet ist.

5. Implantat nach Anspruch 4, bei dem das Abstützteil sich azimutal über mehr als 60°, bevorzugt mehr als 120°, insbesondere 180° oder mehr erstreckt und insbesondere ringförmig gebildet ist.

6. Implantat nach Anspruch 4 oder 5, bei dem das Abstützteil auf einer radial zwischen der Verzahnung und dem Axialdurchgang gelegenen, in der Ebene orthogonal zur Axialrichtung insbesondere kontinuierlichen und insbesondere planar gebildeten Auflage (16) des ersten Stützteils aufliegt.

7. Implantat nach Anspruch 6, mit einem Ringspalt zwischen der Verzahnung und der Auflage.

8. Implantat nach einem der vorhergehenden Ansprüche, mit einer in einem dem ersten Stützteil zugewandten axialen Endbereich des Innengewindes des zweiten Stützteils das Innengewinde sperrenden Axialsicherung (40).

9. Implantat nach einem der vorhergehenden Ansprüche, bei dem das erste Stützteil und/oder das zweite Stützteil insbesondere plattenförmige freie axiale Endbereiche (19, 29) haben, deren Flächenausdehnung im Querschnitt gesehen die des Innengewindes um mehr als 10%, bevorzugt mehr als 20%, weiter bevorzugt mehr als 40%, insbesondere mehr als 60% übersteigt.

10. Implantat nach Anspruch 9 bei dem die freien Endbereiche separat gefertigte und insbesondere angeschraubte Bauteile sind.

11. Implantat nach einem der vorhergehenden Ansprüche, bei dem das erste Stützteil im Bereich des radialen Zugangs eine einen vorragenden, insbesondere diesen umschließenden und dabei durch seine Außenkontur einen insbesondere als Drehsperre gebildeten Formsitz (35) für eine Komplementärform eines Bedieninstruments bildenden Vorsprung aufweist.

12. Instrument (300) zur Betätigung eines Implantats nach einem der vorhergehenden Ansprüche, mit einer von Hand betätigbaren ersten Welle (330) mit einer Gegenverzahnung (336) zu der Verzahnung (36) des Antriebsteils (30) an ihrem proximalen Ende, sowie mit einer Hülsenanordnung (340, 330), durch die die erste Radialwelle führbar ist, und einer zweiten Radialwelle (340) mit einer axial lösbaren drehfesten Kopplung (345) an die Axialsicherung (60), wobei auch die Axialsicherung durch das Innere der Hülsenanordnung passt.

13. Instrument nach Anspruch 12, bei dem die Hülsenanordnung eine Außenhülse mit an ihrem proximalen Ende Komplementärform zum Formsitz des Vorsprungs und eine Innenhülse mit einem Gewinde zum Verschrauben mit einem Innengewinde der Zugangsöffnung aufweist.

14. Set, aufweisend ein oder mehrere Implantate nach einem der Ansprüche 1 bis 11 und einem Instrument (300) zur Betätigung eines Implantats nach einem der Ansprüche 1 bis 11, mit einer von Hand betätigbaren ersten Welle (330) mit einer Gegenverzahnung (336) zu der Verzahnung (36) des Antriebsteils (30) an ihrem proximalen Ende.

15. Set nach Anspruch 14, aufweisend mehrere Implantate mit wenigstens zwei oder mehr unterschiedlichen Axialabmessungen der Implantate bei Einstellung auf deren geringste einstellbare Höhe.

## Claims

1. An implant (200), in particular for supporting the spine, that is height adjustable by means of a rotation, around an axial axis (X), of a drive part that is braced axially relative to a first support part (10) and provided with an external thread (32) cooperating with an internal thread (22) of a second support part (20) that is axially mobile relative to the first support part but rotationally immobile; wherein the drive part has, on an axial side thereof facing the first support part, teeth (36) that are accessible through an access (14) leading radially through the first support part,
**characterised in that** the bracing of the drive part is provided by a bridge (54) spanning a spatial region located azimuthally, relative to the axial axis (X), between the two sides of the access, and the implant (200) has a bracing region of the bracing which is located azimuthally in the area of the access and radially further inwards than the teeth relative to the axial axis (X), and which has the bridge (54).

2. The implant according to claim 1, wherein an opening region of the access extends as far as an axial passage through an axial region of the first support part through which an axial position (38) coupled in a rotationally fixed manner to the drive part penetrates.

3. The implant according to claim 2, comprising a securing device (60) which passes through the opening region and secures the implant in an adjusted-height state in a secured position that prevents rotation of the drive part relative to the first support part.

4. The implant according to one of claims 1 to 3, wherein the bridge (54) is formed by a bracing part (50) that is separate from the first implant part.

5. The implant according to claim 4, wherein the bracing part extends azimuthally over more than 60°, preferably more than 120°, in particular 180° or more, and in particular is formed as a ring.

6. The implant according to claim 4 or 5, wherein the bracing part sits on a bearing (16) of the first support part, the bearing being located radially between the teeth and the axial passage and being in particular continuous in a plane orthogonal to the axial direction, and in particular formed in a planar fashion.

7. The implant according to claim 6, having an annular gap between the teeth and the bearing.

8. The implant according to one of the preceding claims, comprising an axial securing device (40) that locks the internal thread in an axial end region, facing the first support part, of the internal thread of the second support part.

9. The implant according to one of the preceding claims, wherein the first support part and/or the second support part have free axial end regions (19, 29), especially plate-shaped, with a surface area that, when seen in a cross-section, exceeds that of the internal thread by more than 10%, preferably by more than 20%, further preferably by more than 40%, and in particular by more than 60%.

10. The implant according to claim 9, wherein the free end regions are independently manufactured components that are especially screwed on.

11. The implant according to one of the preceding claims, wherein the first support part has a protruding projection in the region of the radial access which in particular surrounds this access and, through its outer contour, forms a shaped seat (35) for a complementary shape of an operating instrument, with said shaped seat in particular being formed as a rotation lock.

12. An instrument (300) for actuating an implant according to one of the preceding claims, comprising a manually actuatable first shaft (330) having a proximal end with mating teeth (336) for the teeth (36) of the drive part (30), and comprising a sleeve arrangement (340, 330) through which the first radial guide can be guided, and a second radial shaft (340) having an axially releasable, rotationally fixed coupling (345) to the axial securing device (60), wherein the axial securing device also fits through the interior of the sleeve arrangement.

13. The instrument according to claim 12, wherein the sleeve arrangement has an outer sleeve comprising a proximal end with a shape complementary to the shaped seat of the projection, and an inner sleeve having a thread for screwing together with an internal thread of the access opening.

14. A set comprising: one or more implants according to one of claims 1 to 11; and an instrument (300) for actuating an implant according to one of claims 1 to 11, comprising a manually actuatable first shaft (330) having a proximal end with mating teeth (336) for the teeth (36) of the drive part (30).

15. The set according to claim 14, comprising a plurality of implants having a minimum of two or more different axial dimensions of the implants when adjusted to their smallest adjustable height.

## Revendications

1. Implant (200), notamment pour le soutien de la colonne vertébrale, doté d'un réglage en hauteur s'effectuant par rotation, autour d'un axe longitudinal (X), d'une pièce d'actionnement supportée axialement par rapport à une première pièce de support (10) et dotée d'un filetage externe (32) coopérant avec un filetage interne (22) d'une deuxième pièce de support (20) mobile axialement par rapport à la première pièce de support mais non mobile en rotation, laquelle pièce d'actionnement est également pourvue, sur sa face axiale tournée vers la première pièce de support, d'une denture (36) accessible par un accès (14) traversant radialement la première pièce de support ;
**caractérisé en ce que** la pièce d'actionnement est supportée par un pont (54) s'étendant sur une zone spatiale située azimutalement par rapport à l'axe longitudinal (X) entre les deux côtés de l'accès, et l'implant (200) présente une zone de support du support qui est située azimutalement dans la zone de l'accès et radialement plus à l'intérieur que la denture par rapport à l'axe longitudinal (X) et qui présente le pont (54).

2. Implant selon la revendication 1, dans lequel une zone d'ouverture de l'accès s'étend jusqu'à un passage axial traversant une zone axiale de la première pièce de support, qui est traversée par une position axiale (38) couplée de manière fixe en rotation à la pièce d'actionnement.

3. Implant selon la revendication 2, comportant un dispositif de blocage (60) traversant la zone d'ouverture et bloquant l'implant, à l'état de hauteur réglée, dans une position de blocage contre une rotation de la pièce d'actionnement par rapport à la première pièce de support.

4. Implant selon l'une des revendications 1 à 3, dans lequel le pont (54) est formé par une pièce de support (50) distincte de la première pièce d'implant.

5. Implant selon la revendication 4, dans lequel la pièce de support s'étend azimutalement sur plus de 60°, de préférence plus de 120°, notamment 180° ou plus, et est notamment de forme annulaire.

6. Implant selon la revendication 4 ou 5, dans lequel la pièce de support repose sur une surface d'appui (16) de la première pièce de support, laquelle surface d'appui est située radialement entre la denture et le passage axial, en étant notamment continue dans le plan orthogonal au sens axial et de forme notamment plane.

7. Implant selon la revendication 6, comportant un espace annulaire entre la denture et la surface d'appui.

8. Implant selon l'une des revendications précédentes, comportant un dispositif de blocage axial (40) qui verrouille le filetage intérieur dans une zone terminale axiale du filetage intérieur de la deuxième pièce de support qui est tournée vers la première pièce de support.

9. Implant selon l'une des revendications précédentes, dans lequel la première pièce de support et/ou la deuxième pièce de support présentent des zones terminales axiales libres (19, 29) notamment en forme de plaque, dont la superficie, vue en coupe, est supérieure à celle du filetage intérieur de plus de 10 %, de préférence de plus de 20 %, plus préférentiellement de plus de 40 %, notamment de plus de 60 %.

10. Implant selon la revendication 9, dans lequel les zones terminales libres sont des composants fabriqués séparément qui sont notamment vissés.

11. Implant selon l'une des revendications précédentes, dans lequel la première pièce de support présente, dans la zone de l'accès radial, une saillie qui entoure notamment cette dernière et qui forme ainsi, par son contour extérieur, un siège profilé (35) constituant notamment un verrou de rotation pour une forme complémentaire d'un instrument connexe.

12. Instrument (300) pour actionner un implant selon l'une des revendications précédentes, comprenant un premier arbre (330) actionnable à la main et pourvu d'une contre-denture (336) à la denture (36) de la pièce d'actionnement (30) à son extrémité proximale, et comprenant un ensemble tubulaire (340, 330) à travers lequel le premier arbre radial peut être guidé, ainsi qu'un deuxième arbre radial (340) pourvu d'un accouplement (345) - amovible axialement et fixe en rotation - au dispositif de blocage (60) axial, ledit dispositif de blocage axial passant également par l'intérieur de l'ensemble tubulaire.

13. Instrument selon la revendication 12, dans lequel l'ensemble tubulaire comprend un tube extérieur présentant, à son extrémité proximale, une forme complémentaire au siège profilé de la saillie, et un tube intérieur présentant un filetage permettant un vissage sur un filetage interne de l'ouverture d'accès.

14. Ensemble comprenant un ou plusieurs implants selon l'une des revendications 1 à 11 et un instrument (300) d'actionnement d'un implant selon l'une des revendications 1 à 11, comprenant un premier arbre (330) actionnable à la main et pourvu d'une contre-denture (336) à la denture (36) de la pièce d'actionnement (30) à son extrémité proximale.

15. Ensemble selon la revendication 14, comprenant plusieurs implants présentant au moins deux dimensions axiales d'implants différentes lorsqu'ils sont réglés à leur hauteur réglable la plus petite.
